# EUROPEAN PATENT APPLICATION

(11) **EP 1 157 999 A1**
(43) Date of publication of application: **28.11.2001**
(21) Application number: 00201837.2
(22) Date of filing: 24.05.2000
(51) Int. Cl.: C07K 14/075, C07K 14/47, C12N 15/86, C12N 5/10, A61K 48/00, A61L 27/60

(54) **Methods and means for enhancing skin transplantation using gene delivery vehicles having tropism for primary fibroblasts, as well as other uses thereof**

(71) Applicant: Introgene B.V., 2333 AL Leiden (NL)
(72) Inventor: Havenga, Menzo Jans Emco, 2401 KG Alphen aan den Rijn (NL); Bout, Abraham, 2751 XL Moerkapelle (NL)
(74) Representative: Prins, Adrianus Willem

(57) **Abstract**

The present invention relates to providing humen primary fibroblasts with a nucleic acid of interest with, among others, the purpose to improve the taking of e.g. skin transplants. The invention particularly relates to transducing fibroblasts with said nucleic acid of interest by means of gene delivery vehicles, in particular chimaeric recombinant adenovirus (having an improved tropism for human primary fibroblasts) based gene delivery vehicles. The present invention is exemplified by an adenovirus serotype 5 genome based vector with an adenoviral fiber protein of a B-type or a D-type adenovirus, in particular adenovirus types 40 or 16, and wherein the nucleic acid of interest encodes a protein which improves angiogenesis and/or neurovascularisation, in particular myogenin or MyoD.

## Description

The present invention relates to providing primary fibroblasts with a nucleic acid of interest with, among others, the purpose to improve the taking of e.g. skin transplants. The invention particularly relates to transducing fibroblasts with said nucleic acid of interest by means of gene delivery vehicles, in particular adenovirus based gene delivery vehicles.
On a yearly basis, millions of people, worldwide, suffer from severe skin burns or chronic skin ulcers caused by pressure, venous status or diabetes mellitus (Brigham et al, 1996). Normal wound healing is a process that can be divided, schematically, into 3 phases (reviewed in Singer et al 1999) 1) inflammation, 2) tissue formation, and 3) tissue remodeling. In the first phase, recruitment of platelets, leukocytes, neutrophils, and monocytes to the site of injury is pivotal to ensure the formation of an extracellular matrix that allows cell migration, cleansing of the wound of foreign particles, i.e. viruses, bacteria, and the initiation and propagation of new tissue formation. Factors involved in initial recruitment of cells to a site of injury are: platelet derived growth factor (PDGF), transforming growth factor alpha and beta 1, 2, or 3 (TGF-α, TGF-β1-2-3), fibroblast growth factor (FGF), Vascular endothelial growth factor (VEGF), insulin-like growth factor (IGF), or keratinocyte growth factor. Furthermore, other vasoactive and chemotactic factors secreted by injured parenchymal cells may play a role as well. In the second phase, epidermal cells at the wound margin start to proliferate into the wound space thereby degrading the extracellular matrix. Factors involved in these processes are collagenase I, stromelysin 1, gelatinase A, collagenase 3 (matrix metalloproteinase 1, 3, 2, and 13) and plasmin produced by plasminogen activator (u-PA). Proliferation of epidermal cells is driven by the release of many factors including epidermal growth factor (EGF), transforming growth factor alpha (TGF-α), and keratinocyte growth factor (Pilcher et al, 1997; Bugge et al 19996; Mignatti et al 1996).
One critical factor in the process of re-epithelialization is neo-vascularization since new blood vessels are required for the nutrient supply to the newly formed tissue. The induction of angiogenesis is a complex process involving many different molecules such as VEGF, TGF-β, angiogenin, angiotropin, angiopoietin 1, thrombospondin, (Folkman et al 1996; Iruela-Arispe et al 1997; Risau 1997) and perhaps constitutively expressed nitric oxide synthase (ceNOS, ecNOS, NOSIII). The third phase involves the remodeling of collagen, appearance of myofibroblasts, wound contraction, and connective tissue compaction. In this phase of woundhealing again metalloproteinases, inhibitors of metalloproteinases play a crucial role. All in all, wound healing is a complex process involving many different steps, cell types, and stimulatory or inhibitory molecules. Abnormal healing of skin can be caused on any level described above. For instance, abnormalities in cell migration, proliferation, inflammation, synthesis and secretion of extracellular matrix proteins and cytokines, remodeling of wound matrix, increased activity of fibrogenic cytokines and exaggerated responses to these cytokines, mutations in regulatory genes such as p53, and abnormal epidermal-mesenchymal interactions have been reported (Fahey et al 1991; Loots, 1998, Tredget et al 1997; Babu et al, 1992, Zhang et al, 1995; Saed et al, 1998; Machesney et al, 1998).
For treatment of severe burn injuries or skin ulcers wound coverage is most important. Also, for plastic surgery, e.g. of the face, long-lasting skin grafts are required.

Therefore, a variety of skin substitutes have been proposed each with its own advantages and disadvantages (see table 2). A major breakthrough in the field of tissue engineering of artificial skin has been the observation that there is no rejection of skin transplants using allogeneic epidermal cells. The lack of rejection is attributed to the absence of MHC-class II HLA-DR antigen expression on epidermal cells (Hefton et al 1983) and the absence of langerhans cells, the antigen presenting cells in the dermis (Thivolet et al, 1986). Thus, it is feasible to generate artificial skin for transplantation purposes to patients suffering from acute or chronic skin ulcers. The ingredients for the formation of artificial skin thus are a matrix providing attachment, fibroblasts and keratinocytes. The material of the matrix being collagen based dermal lattice. Since cultured epiderma-cell allografts are eventually replaced by host cells, their use is thought to be limited to temporary coverage of burns, skin-graft donor sites, and chronic open wounds such as pressure sores and venous stasis ulcers. Thus, it is thought that, unlike autologous grafts, allogeneic artificial skin is inappropriate for permanent coverage of full-thickness wounds. One of the reasons for the temporary use of artificial skin is the lack of take by the host. The grafting percentage of allogeneic artificial skin greatly depends on the material of which the matrix is constructed since the matrix may impose too great a diffusion barrier for the cultured cell graft to become vascularized. Unfortunately, regardless of the material tested today, neovascularization of the skin graft is the most important factor limiting the use of allogeneic artificial skin transplantation. The present invention now provides a solution for this limitation in that it provides methods and means to transduce human fibroblasts with viruses carrying genes encoding for proteins that promote angiogenesis such as described earlier. Preferably, the virus does not integrate into the host-cell genome since the desired effect should be transient only. As a consequence, adenovirus is the gene delivery vehicle of choice.

Gene-transfer vectors derived from adenoviruses (so-called adenoviral vectors) have a number of features that make them particularly useful for gene transfer. 1) the biology of the adenoviruses is characterized in detail, 2) the adenovirus is not associated with severe human pathology, 3) the virus is extremely efficient in introducing its DNA into the host cell, 4) the virus can infect a wide variety of cells and has a broad host-range, 5) the virus can be produced at high virus titers in large quantities, and 6) the virus can be rendered replication defective by deletion of the early-region 1 (E1) of the viral genome (Brody et al, 1994).

However, there are still drawbacks associated with the use of adenoviral vectors, especially the well investigated serotypes of subgroup C adenoviruses. These serotypes require the presence of the Coxackie adenovirus receptor (CAR) on cells for successful infection. Although this protein is expressed by many cells and established cell lines, this protein is absent on many other primary cells and cell lines making the latter cells difficult to infect with serotypes 1, 2, 5, and 6. Also pre-existing immunity and/or the immune response raised aginst the well known adenoviruses is another drawback.

The adenovirus genome is a linear double-stranded DNA molecule of approximately 36000 base pairs. The adenovirus DNA contains identical Inverted Terminal Repeats (ITR) of approximately 90-140 base pairs with the exact length depending on the serotype. The viral origins of replication are within the ITRs exactly at the genome ends.
Most adenoviral vectors currently used in gene therapy have a deletion in the E1 region, where novel genetic information can be introduced. The E1 deletion renders the recombinant virus replication defective (Levrero et al, 1991). It has been demonstrated extensively that recombinant adenovirus, in particular serotype 5 is suitable for efficient transfer of genes *in vivo* to the liver, the airway epithelium and solid tumors in animal models and human xenografts in immunodeficient mice (Bout, 1996; Blaese *et al*., 1995). Thus, preferred methods for *in vivo* gene transfer into target cells make use of adenoviral vectors as gene delivery vehicles.

At present, six different subgroups of human adenoviruses have been proposed which in total encompasses 51 distinct adenovirus serotypes. Besides these human adenoviruses an extensive number of animal adenoviruses have been identified (see Ishibashi et al, 1983).

A serotype is defined on the basis of its immunological distinctiveness as determined by quantitative neutralization with animal antisera (horse, rabbit). If neutralization shows a certain degree of cross-reaction between two viruses, distinctiveness of serotype is assumed if A) the hemagglutinins are unrelated, as shown by lack of cross-reaction on hemagglutination-inhibition, or B) substantial biophysical/ biochemical differences in DNA exist (Francki et al, 1991). The nine serotypes identified last (42-51) were isolated for the first time from HIV-infected patients (Hierholzer et al 1988; Schnurr et al 1993; De Jong et al 1998). For reasons not well understood, most of such immuno-compromised patients shed adenoviruses that were rarely or never isolated from immuno-competent individuals (Hierholzer et al 1988, 1992; Khoo et al, 1995, De Jong et al, 1998).

The adenovirus serotype 5 is most widely used for gene therapy purposes. Similar to serotypes 2, 4 and 7, serotype 5 has a natural affiliation towards lung epithelia and other respiratory tissues. In contrast, it is known that, for instance, serotypes 40 and 41 have a natural affiliation towards the gastrointestinal tract. For a detailed overview of the disease association of the different adenovirus serotypes see table 1. The natural affiliation of a given serotype towards a specific organ can either be due to a difference in the route of infection i.e. make use of different receptor molecules or internalization pathways. However, it can also be due to the fact that a serotype can infect many tissues/organs but it can only replicate in one organ because of the requirement of certain cellular factors for replication and hence clinical disease. At present it is unknown which of the above mentioned mechanisms is responsible for the observed differences in human disease association. However it is known that different adenovirus serotypes can bind to different receptors due to sequence dissimilarity of the capsid proteins i.e. hexon, penton, and fiber protein. For instance, it has been shown that adenoviruses of subgroup C such as Ad2, and Ad5 bind to different receptors as compared to adenoviruses from subgroup B such as Ad3 (Defer et al, 1990). Likewise, it was demonstrated that receptor specificity could be altered by exchanging the Ad3 with the Ad 5 knob protein, and vice versa (Krasnykh et al, 1996; Stevenson et al, 1995, 1997). The present invention now applies this knowledge to identify gene delivery vehicles which can be put to a novel and inventive use in transducing primary fibroblasts which are used to improve skin grafting and in other medicinal applications.

1. Thus the invention provides the use of a recombinant adenovirus having a tropism for human primary fibroblasts as a vehicle for delivering a nucleic acid of interest to a human primary fibroblast. The tropism for the (human) primary fibroblast may be a tropism that the virus itself exhibits, or preferably a tropism that the virus has been provided with. As mentioned herein before the tropism is at least partially accounted for by the fiber protein of a (recombinant) adenovirus. Typically the adenovirus of the invention will therefor have at least a tropism determining part of a fiber protein of a virus fiber protein having tropism for primary fibroblasts. In this is not the wild type fiber protein of the adenovirus used, the result is a chimaeric adenovirus. The invention thus also provides a use in which said recombinant adenovirus is a chimaeric adenovirus. We have shown a number of fibers, which when grafted in an adenovirus 5 (the sequence encoding the fiber replaces the ad5 seqeunce encoding the fiber), typically fibers or parts thereof derived from group B and/or d viruses to have a higher infection rate of human primary fibroblasts. Thus the invention provides viruses and their use, wherein said tropism is provided by at least a tropism determining part of an adenoviral fiber protein of a B-type or a D-type adenovirus, in particular from an adenovirus type 11, 16, 35, 51, 9, 13, 17, 32 and/or 38. Of course combinations of motifs from these fibers in and Ad5 backbone or in another backbone, in particular one with reduced immunogenicity (in any case a preferred embodiment of the present invention) are also part of the present invention. This also goes for a particular fiber from adenovirus type 40. Thus the invention also provides a virus and its use, wherein said fiber protein is derived from a short fiber protein of an adenovirus type 40. The most preferred virus for transfecting primary fibroblasts is one whereinthe tropism is derived from a fiber protein of an adenovirus type 16 or a functional equivalent and/or homologue thereof. A functional equivalent is an amino acid sequence based on or derived from the fiber 16 sequence, which has the same function (in kind, not necessary in quantity) of providing the said tropism. A homologue is an amino acid sequence derived from a different virus, but having the same or similar function as the fiber 16 sequence. Typically high homology between the equivalent and/or the homologue and the original fiber 16 sequence will exist. One of the objectives of the present invention is to improve methods of skin transplatation (temporarily and/or permanently) by means of gene therapy to primary fibroblasts in a skin graft. For that purpose it is preferred to provide the gene delivery vehicles with a nucleic acid of interest encoding a proteinaceous substance which improves angiogenesis and/or neovascularisation, since this is one of the main problems for such grafts. Combinations of different angiogenic proteins is of course also part of the present invention. A proteinaceous substance is a substance which comprises amino acids linked through a peptide bond, including polypeptides (or proteins), glycosylated polypeptides, proteins comprsing subunits, compexes of proteins with nucleic acids, etc.

The invention also provides the use of gene delivery vehicles according to the invention to transduce primary fibroblasts with nucleic acids of interest for different syndroms. Thus the invention provides a gene delivery vehicle and its use, wherein said nucleic acid of interest is a MyoD gene and/or a Myogenin gene or a functional equivalent thereof. Another area in which efficient adenoviral transduction of primary human fibroblasts is a prerequisite is genetic counseling for couples which are suspected of inherited disease, an example being Duchenne Muscular Dystrophy (DMD). On a yearly basis, thousands of couples request family screening for DMD when this disease is known to run in the family. The cells of choice for testing are primary human fibroblasts obtained from skin biopsies. The primary fibroblasts are expanded and used for PCR-based mutational analysis. In hundreds of cases, PCR-analysis is not conclusive and differentiation of primary fibroblasts to myofibroblasts is required (Roest et al, 1996a; Roest et al 1996b). The myofibroblasts may or may not express the dystrophin protein which can be detected using standard immunohistological methods. To ensure a rapid phenotypic differentiation of fibroblasts to myo fibroblasts, the MyoD gene and/ or the Myogenin gene need to be introduced into fibroblasts. This has proven difficult with Ad5 based adenovirus since only small percentages can be transduced of which only a small percentage differentiates into myofibroblasts presumably due to low levels of expression of the MyoD and/ or Myogenin gene.
Since adenovirus 5 is a well known virus with promising results in the field of gene therapy so far, one of the preferred embodiments of the present invention is to provide a recombinant adenovirus based on type 5, with a nucleic acid of interest inserted therein and the fiber replaced by a fiber (or part thereof) having the desired tropism. Thus the invention also provides a virus and its use wherein said virus comprises an adenovirus 5 nucleic acid sequence, preferably a chimaeric virus having at least a deletion in its E1 region where a nucleic acid of interest is inserted or can be inserted and a deletion in the fiber region which is replaced by a nucleic acid sequence having the desired tropism. Apart from the uses described above the invention also provides the recombinant adenoviruses as described above, herein also designated as gene delivery vehicles. However gene delivery vehicles according to the invention are based on adenovirus (for tropism in particular) but may comprise parts of other viruses, even to the extent that only the tropism is derived from an adenoviral fiber. Preferred are chimeric adenoviruses, which also include chimeras with other viruses such as AAV, retroviruses, etc.
Thus the inventuion provides in a preferred embodiment a gene delivery vehicle for delivering a nucleic acid of interest to a primary fibroblast, comprising a recombinant adenovirus having tropism for a primary fibroblast and a nucleic acid sequence encoding a proteinaceous substance which improves angiogenesis and/or neovascularisation as a nucleic acid of interest. In a further preferred embodiment the invention provides a gene delivery vehicle for delivering a nucleic acid of interest to a primary fibroblast, comprising a recombinant adenovirus having tropism for a primary fibroblast and a MyoD gene and/or a Myogenin gene or a functional equivalent thereof.

Preferably said tropism for the gene delivery vehciles according to the invention is provided by a nucleic acid sequence encoding at least a tropism determining part of a fiber protein of an adenovirus type B and/or adenovirus type D, in particular by a nucleic acid sequence encoding at least a tropism determining part of a fiber protein of an adenovirus type 11, 16, 35, 51, 9, 13, 17, 32 and/or 38.

Most preferred is a gene delivery vehicle wherein said tropism is provided by a nucleic acid sequence encoding at least a tropism determining part of a fiber protein of an adenovirus type 16.
As stated herein before a highly preferred gene delivery vehicle is one wherein said chimaeric virus comprises an adenovirus 5 genome, having at least a deletion in its E1 region where a nucleic acid of interest is inserted or can be inserted and a deletion in the fiber region which is replaced by a nucleic acid sequence having the desired tropism. Preferably further alterations are made to decrease immunogenicity (providing parts of Adenovirus 35 or equivalents thereof).
Cells for e.g. skin allografts are also part of the present invention, which provides a human primary fibroblast transduced with a gene delivery vehicle according to the invention.
Skin allograft comprising a human primary fibroblast described above or offspring thereof are also part of the present invention. The invention further provides a method for improving allogeneic or autologous skin transplantation comprising transducing human primary fibroblasts with a gene delivery vehicle according to the inmvention, preparing a skin graft with said human primary fibroblasts and applying said graft to a patient.
The invention uses a library of adenoviruses in which the sequence encoding for the fiber protein from alternative serotypes has been cloned into an adenovirus serotype 5 backbone thereby generating a chimeric adenovirus. This chimeric adenovirus thus has the host range of the adenovirus serotype of which the fiber sequence was cloned whereas all other aspects are derived from adenovirus serotype 5.
Of course also a gene of interest can be inserted at for instance the site of E1 of the original adenovirus from which the vector is derived. In this manner the chimaeric adenovirus to be produced can be adapted to the requirements and needs of certain hosts in need of gene therapy for certain disorders. Naturally, to enable production of a chimeric adenovirus, a packaging cell will generally be needed in order to produce sufficient amount of safe chimaeric adenoviruses.

An important feature of the present invention is the means to produce the chimaeric virus. Typically, one does not want an adenovirus batch to be administered to a host cell which contains replication competent adenovirus, although this is not always true. In general therefor it is desired to omit a number of genes (but at least one) from the adenoviral genome on the vector encoding the chimaeric virus and to supply these genes in the genome of the cell in which the vector is brought to produce chimaeric adenovirus. Such a cell is usually called a packaging cell. The invention thus also provides a packaging cell for producing a chimaeric adenovirus according to the invention, comprising in trans all elements necessary for adenovirus production not present on the adenoviral vector according to the invention. Typically vector and packaging cell have to be adapted to one another in that they have all the necessary elements, but that they do not have overlapping elements which lead to replication competent virus by recombination.

### Detailed description.

In both non-limiting examples described herein: wound healing and genetic counseling, an adenovirus with cell type specific tropism for fibroblasts is thus desired. In both examples, transient expression is desired making an adenovirus ideally suited.

Next, the steps involved in adenovirus binding as has been elucidated at present will be described. The initial step for successful infection is binding of adenovirus to its target cell, a process mediated through fiber protein. The fiber protein has a trimeric structure (Stouten et al, 1992) with different lengths depending on the virus serotype (Signas et al 1985; Kidd et all 1993). Different serotypes have polypeptides with structurally similar N and C termini, but different middle stem regions. N-terminally, the first 30 aminoacids are involved in anchoring of the fiber to the penton base (Chroboczek et al, 1995), especially the conserved FNPVYP region in the tail (Arnberg et al 1997). The C-terminus, or knob, is responsible for initial interaction with the cellular adenovirus receptor. After this initial binding secondary binding between the capsid penton base and cell-surface integrins is proposed to lead to internalization of viral particles in coated pits and endocytosis (Morgan et al, 1969; Svensson et al, 1984; Varga et al, 1992; Greber et al, 1993; Wickham et al, 1994). Integrins are αβ-heterodimers of which at least 14 α -subunits and 8 β-sububits have been identified (Hynes et al, 1992). The array of integrins expressed in cells is complex and will vary between cell types and cellular environment. Although the knob contains some conserved regions, between serotypes, knob proteins show a high degree of variability, indicating that different adenovirus receptors might exist. For instance, it has been demonstrated that adenoviruses of subgroup C (Ad2, Ad5) and adenoviruses of subgroup B (Ad3) bind to different receptors (Defner et al, 1990). By using baculovirus produced soluble CAR as well as adenovirus serotype 5 knob protein, Roelvink et all concluded via interference studies that all adenovirus serotypes, except serotypes of subgroup B, enter cells via CAR (Roelvink et al, 1998). The latter, if valid limits the complexity of using different serotypes for gene therapy purposes.

Besides the involvement in cell binding, the fiber protein also contains the type specific γ-antigen, which together with the ε-antigen of the hexon determines the serotype specificity. The γ-antigen is localized on the fiber and it is known that it consists of 17 aminoacids (Eiz et al, 1997). The anti-fiber antibodies of the host are therefore directed to the trimeric structure of the knob. To obtain re-directed infection of recombinant adenovirus serotype 5, several approaches have been or still are under investigation. Wickham et al have altered the RGD (Arg, Gly, Asp) motif in the penton base which is believed to be responsible for the αᵥβ₃ and αᵥβ₅ integrin binding to the penton base. They have replaced this RGD motif by another peptide motif which is specific for the α₄β₁ receptor. In this way targeting the adenovirus to a specific target cell could be accomplished (Wickham et al, 1995, 1996). Krasnykh et al have made use of the HI loop available in the knob. This loop is, based on X-ray crystallographics, located on the outside of the knob trimeric structure and therefore is thought not to contribute to the intramolecular interactions in the knob. Insertion of a FLAG coding sequence into the HI loop resulted in targeting of the adenovirus to target cells by using antibodies which recognize both the FLAG epitope and a cellular receptor (Krasnykh et al, 1998). However, complete CAR independent infection was not observed.

It is an object of the present invention to provide a method and means by which an adenovirus can infect primary human fibroblasts. Therefore, the generation of chimaeric adenoviruses based on adenovirus serotype 5 with modified fiber genes is described. For this purpose, two or three plasmids, which together contain the complete adenovirus serotype 5 genome, were constructed. From this plasmid the DNA encoding the adenovirus serotype 5 fiber protein was removed and replaced by linker DNA sequences which facilitate easy cloning. The plasmid in which the native adenovirus serotype 5 fiber sequence was partially removed subsequently served as template for the insertion of DNA encoding for fiber protein derived from different adenovirus serotypes (human or animal). The DNA's derived from the different serotypes were obtained using the polymerase chain reaction technique in combination with (degenerate) oligonucleotides. At the former E1 location in the genome of adenovirus serotype 5, any gene of interest can be cloned. A single transfection procedure of the two or three plasmids together resulted in the formation of a recombinant chimaeric adenovirus. Although successful introduction of changes in the adenovirus serotype 5 fiber and penton-base have been reported by others, the complex structure of knob and the limited knowledge of the precise aminoacids interacting with CAR render such targeting approaches laborious and difficult.
To overcome the limitations described above we used pre-existing adenovirus fibers to maximize the chance of obtaining recombinant adenovirus which can normally assemble in the nucleus of a producer cell and which can be produced on pre-existing packaging cells. By generating a chimeric adenovirus serotype 5 based fiber library containing fiber proteins of all other human adenovirus serotypes, we have developed a technology which enables rapid screening for a recombinant adenoviral vector with preferred infection characteristics for primary human fibroblasts.

In one aspect the invention describes the construction and use of plasmids consisting of distinct parts of adenovirus serotype 5 in which the gene encoding for fiber protein has been replaced with DNA derived from alternative human or animal serotypes. This set of constructs, in total encompassing the complete adenovirus genome, allows for the construction of unique chimeric adenoviruses customized for transduction of particular cell types or organ(s). Also, in this part of the invention means and methods to propagate, produce, and purify fiber chimeric adenoviruses is described.

In another aspect of the invention chimeric viruses are described which have preferred infection characteristics in human primary fibroblasts. The adenoviral vectors preferably are derived from subgroup B adenoviruses or contain at least a functional part of the fiber protein from an adenovirus from subgroup B comprising at least the binding moiety of the fiber protein. In a further preferred embodiment the adenoviral vectors are chimeric vectors based on adenovirus serotype 5 and contain at least a functional part of the fiber protein from adenovirus type 16, 35, or 51. It is to be understood that in all embodiments the adenoviral vectors may be derived from the serotype having the desired properties or that the adenoviral vector is based on an adenovirus from one serotype and contains the sequences comprising the desired functions of another serotype, these sequences replacing the native sequences in the said serotype

In another aspect of the invention the chimeric adenoviruses may, or may not, contain deletions in the E1 region and insertions of heterologous genes linked either or not to a promoter. Furthermore, chimeric adenoviruses may, or may not, contain deletions in the E3 region and insertions of-heterologous genes linked to a promoter. Furthermore, chimeric adenoviruses may, or may not, contain deletions in the E2 and/ or E4 region and insertions of heterologous genes linked to a promoter. In the latter case E2 and/ or E4 complementing cell lines are required to generated recombinant adenoviruses.

### Example 1: Generation of adenovirus serotype 5 genomic plasmid clones

The complete genome of adenovirus serotype 5 has been cloned into various plasmids or cosmids to allow easy modification of parts of the adenovirus serotype 5 genome, while still retaining the capability to produce recombinant virus. For this purpose the following plasmids were generated:

### 1. pBr/Ad.Bam-rITR (ECACC deposit P97082122)

In order to facilitate blunt end cloning of the ITR sequences, wild-type human adenovirus type 5 (Ad5) DNA was treated with Klenow enzyme in the presence of excess dNTPs.

After inactivation of the Klenow enzyme and purification by phenol/chloroform extraction followed by ethanol precipitation, the DNA was digested with BamHI. This DNA preparation was used without further purification in a ligation reaction with pBr322 derived vector DNA prepared as follows: pBr322 DNA was digested with EcoRV and BamHI, dephosphorylated by treatment with TSAP enzyme (Life Technologies) and purified on LMP agarose gel (SeaPlaque GTG). After transformation into competent *E*.*coli* DH5a (Life Techn.) and analysis of ampiciline resistant colonies, one clone was selected that showed a digestion pattern as expected for an insert extending from the BamHI site in Ad5 to the right ITR.
Sequence analysis of the cloning border at the right ITR revealed that the most 3' G residue of the ITR was missing, the remainder of the ITR was found to be correct. Said missing G residue is complemented by the other ITR during replication.

### 2. pBr/Ad.Sal-rITR (ECACC deposit P97082119)

pBr/Ad.Bam-rITR was digested with BamHI and SalI. The vector fragment including the adenovirus insert was isolated in LMP agarose (SeaPlaque GTG) and ligated to a 4.8 kb SalI-BamHI fragment obtained from wt Ad5 DNA and purified with the Geneclean II kit (Bio 101, Inc.). One clone was chosen and the integrity of the Ad5 sequences was determined by restriction enzyme analysis. Clone pBr/Ad.Sal-rITR contains adeno type 5 sequences from the SalI site at bp 16746 up to and including the rITR (missing the most 3' G residue).

### 3. pBr/Ad.Cla-Bam (ECACC deposit P97082117)

wt Adeno type 5 DNA was digested with ClaI and BamHI, and the 20.6 kb fragment was isolated from gel by electro-elution. pBr322 was digested with the same enzymes and purified from agarose gel by Geneclean. Both fragments were ligated and transformed into competent DH5a. The resulting clone pBr/Ad.Cla-Bam was analysed by restriction enzyme digestion and shown to contain an insert with adenovirus sequences from bp 919 to 21566.

### 4. pBr/Ad.AflII-Bam (ECACC deposit P97082114)

Clone pBr/Ad.Cla-Bam was linearized with EcoRI (in pBr322) and partially digested with AflII. After heat inactivation of AflII for 20' at 65°C the fragment ends were filled in with Klenow enzyme. The DNA was then ligated to a blunt double stranded oligo linker containing a PacI site (5'-AATTGTCTTAATTAACCGCTTAA-3'). This linker was made by annealing the following two oligonucleotides: 5'-AATTGTCTTAATTAACCGC-3' and 5'-AATTGCGGTTAATTAAGAC-3', followed by blunting with Klenow enzyme. After precipitation of the ligated DNA to change buffer, the ligations were digested with an excess PacI enzyme to remove concatameres of the oligo. The 22016 bp partial fragment containing Ad5 sequences from bp 3534 up to 21566 and the vector sequences, was isolated in LMP agarose (SeaPlaque GTG), religated and transformed into competent DH5a. One clone that was found to contain the PacI site and that had retained the large adeno fragment was selected and sequenced at the 5' end to verify correct insertion of the PacI linker in the (lost) AflII site.

### 5. pBr/Ad.Bam-rITRpac#2 (ECACC deposit P97082120) and pBr/Ad.Bam-rITR#8 (ECACC deposit P97082121)

To allow insertion of a PacI site near the ITR of Ad5 in clone pBr/Ad.Bam-rITR about 190 nucleotides were removed between the ClaI site in the pBr322 backbone and the start of the ITR sequences. This was done as follows: pBr/Ad.Bam-rITR was digested with ClaI and treated with nuclease Bal31 for varying lengths of time (2', 5', 10' and 15'). The extent of nucleotide removal was followed by separate reactions on pBr322 DNA (also digested at the ClaI site), using identical buffers and conditions. Bal31 enzyme was inactivated by incubation at 75°C for 10 minutes, the DNA was precipitated and resuspended in a smaller volume of TE buffer. To ensure blunt ends, DNAs were further treated with T4 DNA polymerase in the presence of excess dNTPs. After digestion of the (control) pBr322 DNA with SalI, satisfactory degradation (^{∼}150 bp) was observed in the samples treated for 10' or 15'. The 10' or 15' treated pBr/Ad.Bam-rITR samples were then ligated to the above described blunted PacI linkers (See pBr/Ad.AflII-Bam). Ligations were purified by precipitation, digested with excess PacI and separated from the linkers on an LMP agarose gel. After religation, DNAs were transformed into competent DH5a and colonies analyzed. Ten clones were selected that showed a deletion of approximately the desired length and these were further analyzed by T-track sequencing (T7 sequencing kit, Pharmacia Biotech). Two clones were found with the PacI linker inserted just downstream of the rITR. After digestion with PacI, clone#2 has 28 bp and clone #8 has 27 bp attached to the ITR.

### pWE/Ad.AflII-rITR (ECACC deposit P97082116)

Cosmid vector pWE15 (Clontech) was used to clone larger Ad5 inserts. First, a linker containing a unique PacI site was inserted in the EcoRI sites of pWE15 creating pWE.pac. To this end, the double stranded PacI oligo as described for pBr/Ad.AflII-BamHI was used but now with its EcoRI protruding ends. The following fragments were then isolated by electro-elution from agarose gel: pWE.pac digested with PacI, pBr/AflII-Bam digested with PacI and BamHI and pBr/Ad.Bam-rITR#2 digested with BamHI and PacI. These fragments were ligated together and packaged using 1 phage packaging extracts (Stratagene) according to the manufacturer's protocol. After infection into host bacteria, colonies were grown on plates and analyzed for presence of the complete insert. pWE/Ad.AflII-rITR contains all adenovirus type 5 sequences from bp 3534 (AflII site) up to and including the right ITR (missing the most 3' G residue).

### pBr/Ad.lITR-Sal(9.4) (ECACC deposit P97082115)

Adeno 5 wt DNA was treated with Klenow enzyme in the presence of excess dNTPs and subsequently digested with SalI. Two of the resulting fragments, designated left ITR-Sal(9.4) and Sal(16.7)-right ITR, respectively, were isolated in LMP agarose (Seaplaque GTG). pBr322 DNA was digested with EcoRV and SalI and treated with phosphatase (Life Technologies). The vector fragment was isolated using the Geneclean method (BIO 101, Inc.) and ligated to the Ad5 SalI fragments. Only the ligation with the 9.4 kb fragment gave colonies with an insert. After analysis and sequencing of the cloning border a clone was chosen that contained the full ITR sequence and extended to the SalI site at bp 9462.

### pBr/Ad.lITR-Sal(16.7) (ECACC deposit P97082118)

pBr/Ad.lITR-Sal(9.4) is digested with SalI and dephosphorylated (TSAP, Life Technologies). To extend this clone upto the third SalI site in Ad5, pBr/Ad.Cla-Bam was linearized with BamHI and partially digested with SalI. A 7.3 kb SalI fragment containing adenovirus sequences from 9462-16746 was isolated in LMP agarose gel and ligated to the SalI-digested pBr/Ad.lITR-Sal(9.4) vector fragment.

### pWE/Ad.AflII-EcoRI

pWE.pac was digested with ClaI and 5' protruding ends were filled using Klenow enzyme. The DNA was then digested with PacI and isolated from agarose gel. pWE/AflII-rITR was digested with EcoRI and after treatment with Klenow enzyme digested with PacI. The large 24 kb fragment containing the adenoviral sequences was isolated from agarose gel and ligated to the ClaI-digested and blunted pWE.pac vector using the Ligation Express^{tm} kit from Clontech. After transformation of Ultracompetent XL10-Gold cells from Stratagene, clones were identified that contained the expected insert. pWE/AflII-EcoRI containes Ad5 sequences from bp 3534-27336.

### Construction of new adapter plasmids

The absence of sequence overlap between the recombinant adenovirus and E1 sequences in the packaging cell line is essential for safe, RCA-free generation and propagation of new recombinant viruses. The adapter plasmid pMLPI.TK is an example of an adapter plasmid designed for use according to the invention in combination with the improved packaging cell lines of the invention. This plasmid was used as the starting material to make a new vector in which nucleic acid molecules comprising specific promoter and gene sequences can be easily exchanged.
First, a PCR fragment was generated from pZipΔMo+PyF101(N⁻) template DNA (described in PCT/NL96/00195) with the following primers: LTR-1: 5'-CTG TAC GTA CCA GTG CAC TGG CCT AGG CAT GGA AAA ATA CAT AAC TG-3' and LTR-2: 5'-GCG GAT CCT TCG AAC CAT GGT AAG CTT GGT ACC GCT AGC GTT AAC CGG GCG ACT CAG TCA ATC G-3'. Pwo DNA polymerase (Boehringer Mannheim) was used according to manufacturers protocol with the following temperature cycles: once 5' at 95°C; 3' at 55°C; and 1' at 72°C, and 30 cycles of 1' at 95°C, 1' at 60°C, 1' at 72°C, followed by once 10' at 72°C. The PCR product was then digested with BamHI and ligated into pMLP10 (Levrero *et al*., 1991) vector digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Next, the coding region of the murine HSA gene was inserted. pLTR10 was digested with BstBI followed by Klenow treatment and digestion with NcoI. The HSA gene was obtained by PCR amplification on pUC18-HSA (Kay *et al*., 1990) using the following primers: HSA1, 5'-GCG CCA CCA TGG GCA GAG CGA TGG TGG C-3' and HSA2, 5'-GTT AGA TCT AAG CTT GTC GAC ATC GAT CTA CTA ACA GTA GAG ATG TAG AA-3'. The 269 bp amplified fragment was subcloned in a shuttle vector using the NcoI and BglII sites. Sequencing confirmed incorporation of the correct coding sequence of the HSA gene, but with an extra TAG insertion directly following the TAG stop codon.The coding region of the HSA gene, including the TAG duplication was then excised as a NcoI (sticky)-SalI (blunt) fragment and cloned into the 3.5 kb NcoI(sticky)/BstBI(blunt) fragment from pLTR10, resulting in pLTR-HSA10.
Finally, pLTR-HSA10 was digested with EcoRI and BamHI after which the fragment containing the left ITR, packaging signal, L420 promoter and HSA gene was inserted into vector pMLPI.TK digested with the same enzymes and thereby replacing the promoter and gene sequences. This resulted in the new adapter plasmid pAd/L420-HSA that contains convenient recognition sites for various restriction enzymes around the promoter and gene sequences. SnaBI and AvrII can be combined with HpaI, NheI, KpnI, HindIII to exchange promoter sequences, while the latter sites can be combined with the ClaI or BamHI sites 3' from HSA coding region to replace genes in this construct.
Another adapter plasmid that was designed to allow easy exchange of nucleic acid molecules was made by replacing the promoter, gene and poly A sequences in pAd/L420-HSA with the CMV promoter, a multiple cloning site, an intron and a poly-A signal. For this purpose, pAd/L420-HSA was digested with AvrII and BglII followed by treatment with Klenow to obtain blunt ends. The 5.1 kb fragment with pBr322 vector and adenoviral sequences was isolated and ligated to a blunt 1570 bp fragment from pcDNAl/amp (Invitrogen) obtained by digestion with HhaI and AvrII followed by treatment with T4 DNA polymerase. This adapter plasmid was named pCLIP.

### Generation of recombinant adenoviruses

To generate E1 deleted recombinant adenoviruses with the new plasmid-based system, the following constructs are prepared:
a) An adapter construct containing the expression cassette with the gene of interest linearised with a restriction enzyme that cuts at the 3' side of the overlapping adenoviral genome fragment, preferably not containing any pBr322 vector sequences, and
b) A complementing adenoviral genome construct pWE/Ad.AfIII-rITR digested with PacI.
These two DNA molecules are further purified by phenol/ chloroform extraction and EtOH precipitation. Co-transfection of these plasmids into an adenovirus packaging cell line, preferably a cell line according to the invention, generates recombinant replication deficient adenoviruses by a one-step homologous recombination between the adapter and the complementing construct.
Alternatively, in stead of pWE/Ad.AflII-rITR other fragments can be used, e.g., pBr/Ad.Cla-Bam digested with EcoRI and BamHI or pBr/Ad.AflII-BamHI digested with PacI and BamHI can be combined with pBr/Ad.Sal-rITR digested with SalI. In this case, three plasmids are combined and two homologous recombinations are needed to obtain a recombinant adenovirus. It is to be understood that those skilled in the art may use other combinations of adapter and complementing plasmids without departing from the present invention.
A general protocol as outlined below and meant as a non-limiting example of the present invention has been performed to produce several recombinant adenoviruses using various adapter plasmids and the Ad.AflII-rITR fragment. Adenovirus packaging cells (PER.C6) were seeded in ^{~}25 cm² flasks and the next day when they were at ^{~}80% confluency, transfected with a mixture of DNA and lipofectamine agent (Life Techn.) as described by the manufacturer. Routinely, 40 *µ*l lipofectamine, 4 *µ*g adapter plasmid and 4 *µ*g of the complementing adenovirus genome fragment AflII- rITR (or 2 *µ*g of all three plasmids for the double homologous recombination) are used. Under these conditions transient transfection efficiencies of ^{∼}50% (48 hrs post transfection) are obtained as determined with control transfections using a pAd/CMV-LacZ adapter. Two days later, cells are passaged to ^{∼}80 cm² flasks and further cultured. Approximately five (for the single homologous recombination) to eleven days (for the double homologous recombination) later a cytopathogenic effect (CPE) is seen, indicating that functional adenovirus has formed. Cells and medium are harvested upon full CPE and recombinant virus is released by freeze-thawing. An extra amplification step in an 80 cm² flask is routinely performed to increase the yield since at the initial stage the titers are found to be variable despite the occurrence of full CPE. After amplification, viruses are harvested and plaque purified on PER.C6 cells. Individual plaques are tested for viruses with active transgenes.

Besides replacements in the E1 region it is possible to delete or replace (part of) the E3 region in the adenovirus because E3 functions are not necessary for the replication, packaging and infection of the (recombinant) virus. This creates the opportunity to use a larger insert or to insert more than one gene without exceeding the maximum package size (approximately 105% of wt genome length). This can be done, e.g., by deleting part of the E3 region in the pBr/Ad.Bam-rITR clone by digestion with XbaI and religation. This removes Ad5 wt sequences 28592-30470 including all known E3 coding regions. Another example is the precise replacement of the coding region of gp19K in the E3 region with a polylinker allowing insertion of new sequences. This, 1) leaves all other coding regions intact and 2) obviates the need for a heterologous promoter since the transgene is driven by the E3 promoter and pA sequences, leaving more space for coding sequences.
To this end, the 2.7 kb EcoRI fragment from wt Ad5 containing the 5' part of the E3 region was cloned into the EcoRI site of pBluescript (KS⁻) (Stratagene). Next, the HindIII site in the polylinker was removed by digestion with EcoRV and HincII and subsequent religation. The resulting clone pBS.Eco-Eco/ad5DHIII was used to delete the gp19K coding region. Primers 1 (5'-GGG TAT TAG GCC AA AGG CGC A-3') and 2 (5'-GAT CCC ATG GAA GCT TGG GTG GCG ACC CCA GCG-3') were used to amplify a sequence from pBS.Eco-Eco/Ad5DHIII corresponding to sequences 28511 to 28734 in wt Ad5 DNA. Primers 3 (5'-GAT CCC ATG GGG ATC CTT TAC TAA GTT ACA AAG CTA-3') and 4 (5'-GTC GCT GTA GTT GGA CTG G-3') were used on the same DNA to amplify Ad5 sequences from 29217 to 29476. The two resulting PCR fragments were ligated together by virtue of the new introduced NcoI site and subsequently digested with XbaI and MunI. This fragment was then ligated into the pBS.Eco-Eco/ad5ΔHIII vector that was digested with XbaI (partially) and MunI generating pBS.Eco-Eco/ad5ΔHIII.Δgp19K. To allow insertion of foreign genes into the HindIII and BamHI site, an XbaI deletion was made in pBS.Eco-Eco/ad5 ΔHIII.Δgp19K to remove the BamHI site in the Bluescript polylinker. The resulting plasmid pBS.Eco-Eco/ad5ΔHIIIΔgp19K ΔXbaI, containes unique HindIII and BamHI sites corresponding to sequences 28733 (HindIII) and 29218 (BamHI) in Ad5. After introduction of a foreign gene into these sites, either the deleted XbaI fragment is re-introduced, or the insert is recloned into pBS.Eco-Eco/ad5ΔHIII.Δgpl9K using HindIII and for example MunI. Using this procedure, we have generated plasmids expressing HSV-TK, hIL-1a, rat IL-3, luciferase or LacZ. The unique SrfI and NotI sites in the pBS.Eco-Eco/ad5ΔHIII.Δgpl9K plasmid (with or without inserted gene of interest) are used to transfer the region comprising the gene of interest into the corresponding region of pBr/Ad.Bam-rITR, yielding construct pBr/Ad.Bam-rITRΔgp19K (with or without inserted gene of interest). This construct is used as described *supra* to produce recombinant adenoviruses. In the viral context, expression of inserted genes is driven by the adenovirus E3 promoter.
Recombinant viruses that are both E1 and E3 deleted are generated by a double homologous recombination procedure as described above for E1-replacement vectors using a plasmid-based system consisting of:
a) an adapter plasmid for E1 replacement according to the invention, with or without insertion of a first gene of interest,
b) the pWE/Ad.AflII-EcoRI fragment, and
c) the pBr/Ad.Bam-rITRΔgp19K plasmid with or without insertion of a second gene of interest.
In addition to manipulations in the E3 region, changes of (parts of) the E4 region can be accomplished easily in pBr/Ad.Bam-rITR. Generation and propagation of such a virus, however, in some cases demands complementation *in trans.*

### Example 2: Generation of adenovirus serotype 5 based viruses with chimeric fiber proteins

The method described *infra* to generate recombinant adenoviruses by co-transfection of two, or more seperate cloned adenovirus sequences. One of these cloned adenovirus sequences was modified such that the adenovirus serotype 5 fiber DNA was deleted and substituted for unique restriction sites thereby generating "template clones" which allow for the easy introduction of DNA sequences encoding for fiber protein derived from other adenovirus serotypes.

### Generation of adenovirus template clones lacking DNA encoding for fiber

The fiber coding sequence of adenovirus serotype 5 is located between nucleotides 31042 and 32787. To remove the adenovirus serotype 5 DNA encoding fiber we started with construct pBr/Ad.Bam-rITR. First a NdeI site was removed from this construct. For this purpose, pBr322 plasmid DNA was digested with NdeI after which protruding ends were filled using Klenow enzym. This pBr322 plasmid was then religated, digested with NdeI and transformed into *E*.*coli* DH5α . The obtained pBr/ΔNdeI plasmid was digested with ScaI and SalI and the resulting 3198 bp vector fragment was ligated to the 15349 bp ScaI-SalI fragment derived from pBr/Ad.BamrITR, resulting in plasmid pBr/Ad.Bam-rITRΔNdeI which hence contained a unique NdeI site. Next a PCR was performed with oligonucleotides NY-up: 5'- CGA **CAT ATG** TAG ATG CAT TAG TTT GTG TTA TGT TTC AAC GTG-3'
And NY-down:5'-GGA GAC CAC TGC CAT GTT-3'. During amplification, both a NdeI (bold face) and a NsiI restriction site (underlined) were introduced to facilitate cloning of the amplified fiber DNAs. Amplification consisted of 25 cycles of each 45 sec. at 94°C, 1 min. at 60°C, and 45 sec. at 72°C. The PCR reaction contained 25 pmol of oligonucleotides NY-up or NY-down, 2mM dNTP, PCR buffer with 1.5 mM MgCl₂, and 1 unit of Elongase heat stable polymerase (Gibco, The Netherlands). One-tenth of the PCR product was run on an agarose gel which demonstrated that the expected DNA fragment of ± 2200 bp was amplified. This PCR fragment was subsequently purified using Geneclean kit system (Bio101 Inc.). Then, both the construct pBr/Ad.Bam-rITRΔNdeI as well as the PCR product were digested with restriction enzymes NdeI and SbfI. The PCR fragment was subsequently cloned using T4 ligase enzyme into the NdeI and SbfI digested pBr/Ad.Bam-rITRΔNdeI, generating pBr/Ad.BamRΔFib. This plasmid allows insertion of any PCR amplified fiber sequence through the unique NdeI and NsiI sites that are inserted in place of the removed fiber sequence. Viruses can be generated by a double homologous recombination in packaging cells described *infra* using an adapter plasmid, construct pBr/Ad.AflII-EcoRI digested with PacI and EcoRI and a pBr/Ad.BamRΔFib construct in which heterologous fiber sequences have been inserted. To increase the efficiency of virus generation, the construct pBr/Ad.BamRΔFib was modified to generate a PacI site flanking the right ITR. Hereto, pBr/Ad.BamRΔFib was digested with AvrII and the 5 kb adenofragment was isolated and introduced into the vector pBr/Ad.Bam-rITR.pac#8 replacing the corresponding AvrII fragment. The resulting construct was named pBr/Ad.BamRΔ Fib.pac. Once a heterologous fiber sequence is introduced in pBr/Ad.BamRΔFib.pac, the fiber modified right hand adenovirus clone may be introduced into a large cosmid clone as described for pWE/Ad.AflII-rITR in example 1. Such a large cosmid clone allows generation of adenovirus by only one homologous recombination making the process extremely efficient.

### Amplification of fiber sequences from adenovirus serotypes

To enable amplification of the DNAs encoding fiber protein derived from alternative serotypes degenerate oligonucleotides were synthesized. For this purpose, first known DNA sequences encoding for fiber protein of alternative serotypes were aligned to identify conserved regions in both the tail-region as well as the knob-region of the fiber protein. From the alignment, which contained the nucleotide sequence of 19 different serotypes representing all 6 subgroups, (degenerate) oligonucleotides were synthesised (see table 2). Also shown in table 2 is the combination of oligonucleotides used to amplify the DNA encoding fiber protein of a specific serotype. The amplification reaction (50 µl) contained 2 mM dNTPs, 25 pmol of each oligonucleotide, standard 1x PCR buffer, 1,5 mM MgCl₂, and 1 Unit Pwo heat stable polymerase (Boehringer) per reaction. The cycler program contained 20 cycles, each consisting of 30 sec. 94°C, 60 sec. 60-64°C, and 120 sec. At 72°C. One-tenth of the PCR product was run on an agarose gel which demonstrated that a DNA fragment was amplified. Of each different template, two independent PCR reactions were performed after which the independent PCR fragments obtained were sequenced to determine the nucleotide sequence. From 11 different serotypes, the nucleotide sequence could be compared to sequences present in genbank. Of all other serotypes, the DNA encoding fiber protein was previously unknown and was therefore aligned with known sequences from other subgroup members to determine homology i.e. sequence divergence. Of the 51 human serotypes known to date, all fiber sequences, except for serotypes 1, 6, 18, and 26, have been amplified and sequenced.

### Generation of fiber chimaeric adenoviral DNA constructs

All amplified fiber DNAs as well as the vector (pBr/Ad.BamRΔ Fib) were digested with NdeI and NsiI. The digested DNAs was subsequently run on a agarose gel after which the fragments were isolated from the gel and purified using the Geneclean kit (Bio101 Inc). The PCR fragments were then cloned into the NdeI and NsiI sites of pBr/AdBamRΔFib, thus generating pBr/AdBamRFibXX (where XX stands for the serotype number of which the fiber DNA was isolated). Sofar the fiber sequence of serotypes 5/ 7/ 8/ 9/ 10/ 11/ 12/ 13/ 14/ 16/ 17/ 19/ 21/ 24/ 27/ 28/ 29/ 30/ 32/ 33/ 34/ 35/ 36/ 37/ 38/ 40-S/ 40-L/ 41-S/ 42/45/ 47/ 49/ 51 have been cloned into pBr/AdBamRFibXX. From pBr/AdBamRFibXX (where XX is 5/ 8/ 9/ 10/ 11/ 13/ 16/ 17/ 24/ 27/ 30/ 32/ 33/ 34/ 35/ 38/ 40-S/ 40-L/ 45/ 47/ 49/ 51) a cosmid clone in pWE/Ad.AflII-rITR (see example 1) was generated to facilitate efficient virus generaion. This cosmid cloning resulted in the formation of construct pWE/Ad.AflII-rITR/FibXX (where XX stands for the serotype number of which the fiber DNA was isolated)

### Generation of recombinant adenovirus chimeric for fiber protein

To generate recombinant Ad 5 virus carrying the fiber of serotype 12, 16, 28, 40-L, 51, and 5, three constructs, pCLIP/luciferase, pWE/AdAflII-Eco and pBr/AdBamrITR.pac/fibXX (XX = 12, 16, 28, 40-L, 51, and 5) were transfected into adenovirus producer cells. To generate recombinant Ad 5 virus carrying the fiber of 5/ 7/ 8/ 9/ 10/ 11/ 12/ 13/ 14/ 16/ 17/ 19/ 21/ 24/ 27/ 28/ 29/ 30/ 32/ 33/ 34/ 35/ 36/ 37/ 38/ 40-S/ 40-L/ 41-S/ 42/45/ 47/ 49/ 51, two contructs pCLIP/luciferase and pWE/Ad.AflII-rITR/FibXX were transfected into adenovirus producer cells.
For transfection, 2 µg of pCLIP/luciferase, and 4 µg of both pWE/AdAflII-Eco and pBr/AdBamrITR.pac/fibXX (or in case of cosmids: 4 µg of pCLIP/luciferase plus 4 µg of pWE/Ad.AflII-rITR/FibXX) were diluted in serum free DMEM to 100 µl total volume. To this DNA suspension 100 µl 1x diluted lipofectamine (Gibco) was added. After 30 minutes at room temperature the DNA-lipofectamine complex solution was added to 2.5 ml of serum-free DMEM which was subsequently added to a T25 cm² tissue culture flask. This flask contained 2x10⁶ PER.C6 cells that were seeded 24-hours prior to transfection. Two hours later, the DNA-lipofectamine complex containing medium was diluted once by the addition of 2.5 ml DMEM supplemented with 20% fetal calf serum. Again 24 hours later the medium was replaced by fresh DMEM supplemented with 10% fetal calf serum. Cells were cultured for 6-8 days, subsequently harvested, and freeze/thawed 3 times. Cellular debri was removed by centrifugation for 5 minutes at 3000 rpm room temperature. Of the supernatant (12.5 ml) 3-5 ml was used to infect again infect PER.C6 cells (T80 cm² tissue culture flasks). This re-infection results in full cytopathogenic effect (CPE) after 5-6 days after which the adenovirus is harvested as described above.

### Example 3: Production, purification, and titration of fiber chimeric adenoviruses

Of the supernatant obtained from transfected PER.C6 cells 10 ml was used to inoculate a 1 liter fermentor which contained 1 - 1.5 x 10⁶ cells/ ml PER.C6 that were specifically adapted to grow in suspension. Three days after inoculation, the cells were harvested and pelleted by centrifugating for 10 min at 1750 rpm at roomtemperature. The chimeric adenoviruses present in the pelleted cells were subsequently extracted and purified using the following downstream proccesing protocol. The pellet was disolved in 50 ml 10 mM NaPO₄⁻ and frozen at -20°C. After thawing at 37°C, 5.6 ml deoxycolate (5% w/v) was added after which the solution was homogenated. The solution was subsequently incubated for 15 minutes at 37°C to completely crack the cells. After homogenizing the solution, 1875 µl (1M) MgCl₂⁻ was added and 5 ml 100% glycerol. After the addition of 375 µl DNAse (10 mg/ ml) the solution was incubated for 30 minutes at 37°C. Celldebri was removed by centrifugation at 1880xg for 30 minutes at roomtemperature without the brake on. The supernatant was subsequently purified from proteins by loading on 10 ml of freon. Upon centrifugation for 15 minutes at 2000 rpm without brake at roomtemperature three bands are visable of which the upper band represents the adenovirus. This band was isolated by pipetting after which it was loaded on a Tris/HCl (1M) buffered caesiumchloride blockgradient (range: 1.2 to 1.4 gr./ml). Upon centrifugation at 21000 rpm for 2.5 hours at 10°C the virus was purified from remaining protein and celldebri since the virus, in contrast to the other components, does not migrate into the 1.4 gr./ ml caesiumchloride solution. The virus band is isolated after which a second purification using a Tris/ HCl (1M) buffered continues gradient of 1.33 gr./ml of caesiumchloride is performed. After virus loading on top of this gradient the virus is centrifuged for 17 hours at 55000 rpm at 10°C. Subsequently the virus band is isolated and after the addition of 30 µl of sucrose (50 w/v) excess caesiumchloride is removed by three rounds of dialysis, each round comprising of 1 hour. For dialysis the virus is transferred to dialysis slides (Slide-a-lizer, cut off 10000 kDa, Pierce, USA). The buffers used for dialysis are PBS which are supplemented with an increasing concentration of sucrose (round 1 to 3: 30 ml, 60 ml, and 150 ml sucrose (50% w/v)/ 1.5 liter PBS, all supplemented with 7.5 ml 2% (w/v) CaMgCl₂). After dialysis, the virus is removed from the slide-a-lizer after which it is aliquoted in portions of 25 and 100 µl upon which the virus is stored at -85°C.
To determine the number of virusparticles per milliliter, 100 µl of the virus batch is run on an high pressure liquid chromatograph (HPLC). The adenovirus is bound to the column (anion exchange) after which it is eluted using a NaCl gradient (range 300-600 mM). By determining the area under the viruspeak the number of virus particles can be calculated. To determine the number of infectious units (IU) per ml present in a virus batch, titrations are performed on 911 cells. For this purpose, 4x10⁴ 911 cells are seeded per well of 96-well plates in rows B, D, and F in a total volume of 100 µl per well. Three hours after seeding the cells are attached to the plastic support after which the medium can be removed. To the cells a volume of 200 µl is added, in duplicate, containing different dilutions of virus (range: 10² times diluted to 2x10⁹). By screening for CPE the highest virus dilution which still renders CPE after 14 days is considered to contain at least one infectious unit. Using this observation, together with the calculated amount of virus volume present in these wells renders the number of infectious units per ml of a given virus batch. The production results i.e. virus particles per ml and IU per ml or those chimeric adenoviruses that were produced,all with the luciferase cDNA as a marker, are shown in table 3.

### Example 4: Expression of receptors for Adenovirus 5 on Primary human fibroblasts

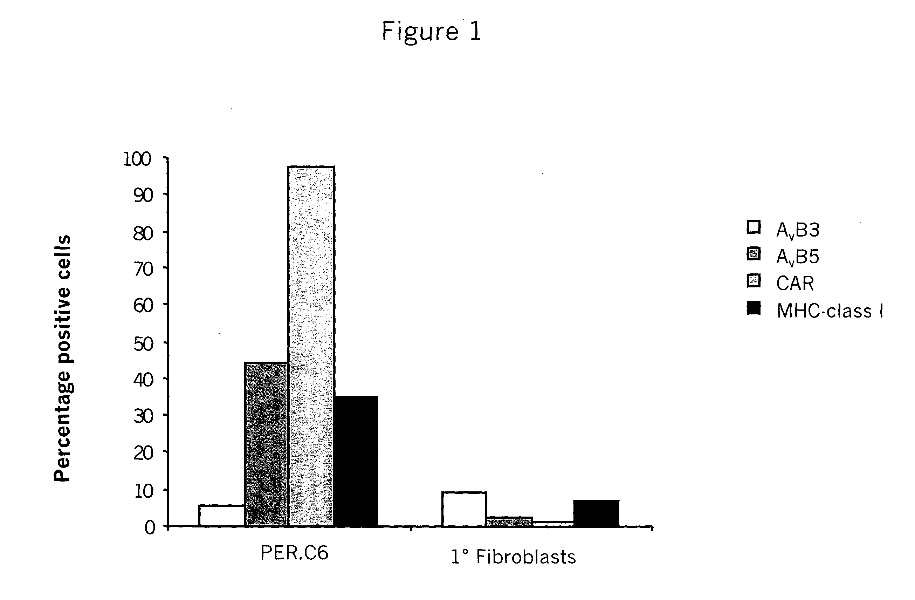

To test whether for expression on primary fibroblasts for membrane molecules known to be involved in Ad5 infection, the presence of CAR, MHC class I, and αᵥ-integrins was assayed on a flow cytometer. For this purpose 1x10⁵ HUVEC cells or HUVsmc were washed once with PBS/ 0.5% BSA after which the cells were pelleted by centrifugation for 5 minutes at 1750 rpm at roomtemperature. Subsequently, 10 µl of a 100 times diluted αᵥβ3 antibody (Mab 1961, Brunswick chemie, Amsterdam, The Netherlands), a 100 times diluted antibody αᵥβ5 (antibody (Mab 1976, Brunswick chemie, Amsterdam, The Netherlands), or 2000 times diluted CAR antibody was a kind gift of Dr. Bergelson, Harvard Medical School, Boston, USA (Hsu et al) was added to the cell pellet after which the cells were incubated for 30 minutes at 4°C in a dark environment. After this incubation, cells were washed twice with PBS/0.5% BSA andagain pelleted by centrifugation for 5 minutes at 1750 rpm roomtemperature. To label the cells, 10 ml of rat anti mouse IgGl labeled with phycoerythrine (PE) was added to the cell pellet upon which the cells were again incubated for 30 minutes at 4°C in a dark environment. Finally the cells were washed twice with PBS/0.5% BSA and analysed on a flow cytometer. The results of these experiments are shown in Figure 1. From the results it can be concluded that primary human fibroblasts do not express detectable levels of CAR or MHC-class I confirming that these cells are difficult to transduce with an adenovirus which enters the cells via these molecules.

### Example 5: Adenovirus transduction of human primary Fibroblasts.

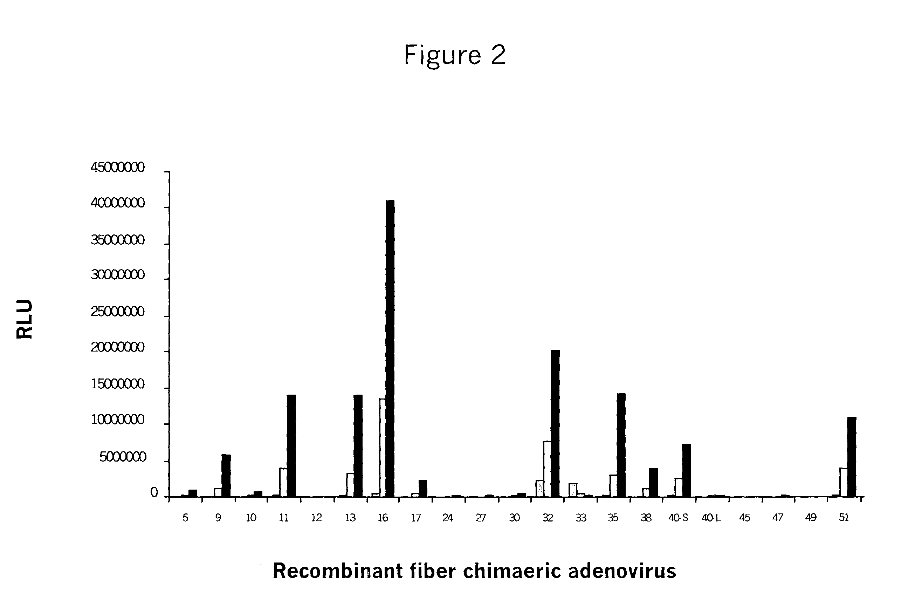

Human primary fibroblasts were rountinely maintained in Dulbecco's modified Eagles medium (DMEM) supplemented with 10% fetal calf serum. Fibroblasts tested were from different origens. Normal human fibroblasts were obtained from Coriell (GM09503), or were isolated from skin biopsis from healthy human individuals.In a first experiment, 10⁵ fibroblasts were seeded in wells of 24-well plates. The next day cells were exposed to either 100, 500, or 1000 virusparticles per cell of recombinant fiber chimaeric viruses carrying the fiber of serotype 9, 10, 11, 12, 13, 16, 17, 24, 27, 30, 32, 33, 35, 38, 40-S, 40-L, 45, 47, 49, or 51. In these experiments, the parent vector (fib5) was taken along as a reference. Forthy-eight hours after the addition of virus, cells were washed twice with 1 ml PBS after which cells were lysed by adding 100 µl of cell lysisbuffer. Lysates were subsequently transferred to 96-well plates and stored at-20°C until luciferase activity measurement. Luciferase activty was determined using a bioluminescence machine, the luciferase assay kit from Promega™ (catalog nr. E-1501) and the instructions provided by the manufacturer. The results of the luciferase transgene expresssion measured in primary human fibroblasts after transduction with the panel of fiber chimeaeric viruses is shown in figure 2. The results demonstrate that Several fiber chimaeric viruses perform better on fibroblasts as compared to the parent vector (Ad5). These viruses carry the fiber from a subgroup B virus i.e. 11, 16, 35, and 51. Also, several, but not all, viruses carrying a fiber originating from subgroup D i.e. 9, 13, 17, 32, 38 seem better equipped for transducing fibroblasts.

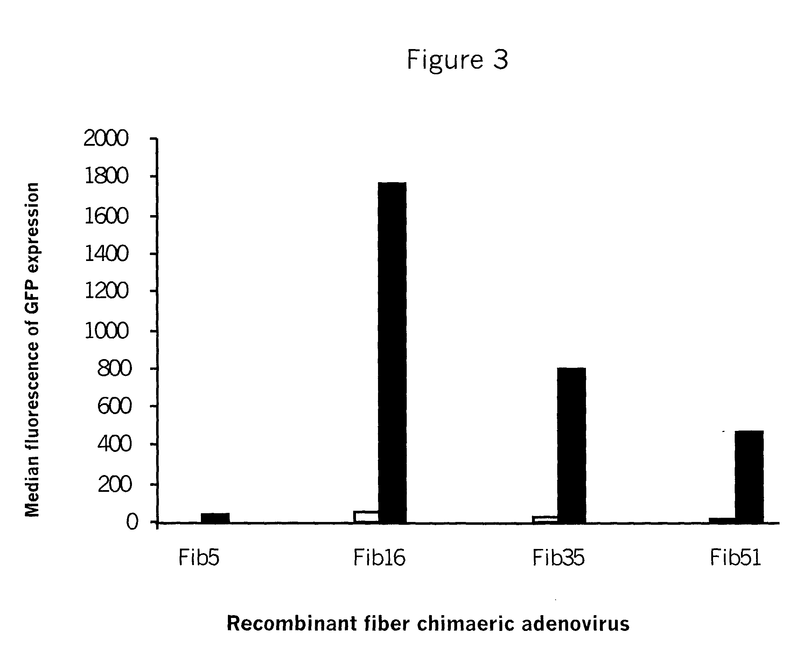

Also, the short fiber of serotype 40 (40-S), a F-group virus performs better than Ad5. Clearly, based on luciferase expression the Ad5 vector carrying a fiber of serotype 16 looked most most promising. In a.next experiment, Ad5 was directly compared to Ad5.Fib16, Ad5.Fib35, and Ad5.Fib51 using green fluorescent protein (GFP) as a marker gene. This marker gene allows single cell analysis using a flow cytometer. Infection of fibroblasts using the GFP viruses was performed identical as described above. The results on GFP expression is shown in figure 3. These results confirm the results of the first experiment for the fiber chimaeric viruses Ad5.Fib16, Ad5.Fib35, and Ad5.Fib51.

### Figure and table legends

Table 1: Association of different human adenovirus serotypes with human disease.
Table 2: Skin substitutes: The manufacturers are as follows: Alloderm: Life cell, Woodlands, Tex; Integra, Life sciences, Plainsboro, N.J.; Dermagraft-TC, Advanced tissue sciences, La Jolla, Calif.; Apligraf, Organogenesis, Canton, Mass.; Composite cultured skin: Ortec international, New York. (taken from Singer et al 1999)
Table 3: Production results of recombinant fiber chimeric adenoviruses. Results in virus particles per milliliter as determined by HPLC.

Figure 1:Expression of CAR, MHC-class I, and av-integrins on primary fibroblasts. As a control for the antibodies Peer.C6 cells were taken along.
Figure 2: Screening the fiber chimaeric viruses for the presence of viruses that are better suited for transduction of primary human fibroblasts. The dose used is 100 (grey bars), 500 (white bars), or 1000 (black bars) virus particles per cell. Luciferase activity is expressed in relative light units (RLU).
Figure 3: Flow cytometric analysis on fibroblasts transduced with 100 (white bars) or 1000 (black bars) virus particles of fiber chimaeric viruses carrying green fluorescent protein as a marker. Shown is the median flourescence obtained after transduction with either Ad5 or the fiber chimaeric viruses carrying the fiber of serotype 16, 35, or 51.

### References

Arnberg N., Mei Y. and Wadell G., 1997. Fiber genes of adenoviruses with tropism for the eye and the genital tract. Virology 227: 239-244.

Babu, M; Diegelman, R; Oliver, N (1992). Keloid fibroblasts exhibit an altered response to TGF-beta. J. Invest. Dermatol. 99, p650-655.

Bergelson, J.M., Cunningham, J.A., Droguett, G., Kurt-Jones, E.A., Krithivas, A., Hong, J.S., Horwitz, M.S., Crowell, R.L. and Finberg, R.W. (1997) Isolation of a common receptor for coxsackie B virus and adenoviruses 2 and 5. Science 275: 1320-1323.

Brigham, PA, McLoughlin, E (1996). Burn incidence and medical care use in the United States: Estimate, Trends, and Data sources. J. Burn Care Rehabil 17, p95-107.

Bout A. (1997) Gene therapy, p. 167-182. In: D.J.A. Crommelin and R.D. Sindelar (ed.), Pharmaceutical Biotechnology , Harwood Academic Publishers.

Bout, A. (1996) Prospects for human gene therapy. Eur. J. Drug Met. and Pharma. 2, 175-179.

Blaese, M., Blankenstein, T., Brenner, M., Cohen-Hagenauer, O., Gansbacher, B., Russel, S., Sorrentino, B. and Velu, T. (1995) Cancer Gene Ther. 2: 291-297.

Bugge, TH; Kombrinck, KW; Flick, MJ; Daugherthy, CC; Danton, MJS; Degen, JL (1996). Loss of fibrinogen rescues mice from the pleiotropic effects of plasminogen deficiency. Cell 87, p709-719.

Brody, S.L. and Crystal, R.G. (1994) Adenovirus mediated in vivo gene transfer. Ann. N. Y. Acad. Sci. 716:90-101.

Chroboczek J., Ruigrok R.W.H., and Cusack S., 1995. Adenovirus fiber, p. 163-200. In: W. Doerfler and P. Bohm (ed.), The molecular repertoire of adenoviruses, I. Springer-Verlag, Berlin.

Defer C., Belin M., Caillet-Boudin M. and Boulanger P., 1990. Human adenovirus-host cell interactions; comparative study with members of subgroup B and C. Journal of Virology 64 (8): 3661-3673.

Fallaux, F.J, Bout, A, van der Velde, I et al. New helper cells and matched E1-deleted adenovirus vectors prevent generation of replication competent adenoviruses. Human Gene Therapy, 9 (1998), p1909-1917.

Fahey, TJ III, Sadaty, A; Jones, WG II, Barber, A; Smoller, B; Shires, GT (1991). Diabetes impairs the late inflammatory response to wound healing. J. Surg. Res 50, p308-313.

Francki, R.I.B., Fauquet, C.M., Knudson, D.L. and Brown, F. (1991) Classification and nomenclature of viruses. Fifth report of the international Committee on taxonomy of viruses. Arch. Virol. Suppl. 2: 140-144.

Folkman, J; D'Amore, PA (1996). Blood vessel formation. What is its molecular basis. Cell 87, p1153-1155.

Gall J., Kass-Eisler A., Leinwand L. and Falck-Pedersen E. (1996) Adenovirus type 5 and 7 capsid chimera: fiber replacement alters receptor tropism without affecting primary immune neutralization epitopes. Journal of Virology 70 (4): 2116-2123.

Greber, U.F., Willets, M., Webster, P., and Helenius, A. (1993). Stepwise dismanteling of adenovirus 2 during entry into cells. Cell 75: 477-486.

Hynes, R.O. (1992) Integrins: versatility, modulation and signalling in cell adhesion. Cell 69: 11-25.

Hefton, JM; Madden, MR; Finkelstein, JM; Shires, GT (1983). Grafting of burn patients with allografts of cultured epidermal cells. Lancet 2, p428-430.

Herz, J. and Gerard, R.D. (1993) Adenovirus-mediated transfer of low density lipoprotein receptor gene acutely accelerates cholesterol clearence in normal mice. Proc. Natl. Acad. Sci. U.S.A. 96: 2812-2816.

Hierholzer, J.C. (1992) Adenovirus in the immunocompromised host. Clin. Microbiol Rev. 5, 262-274.

Hierholzer, J.C., Wigand, R., Anderson, L.J., Adrian, T., and Gold, J.W.M. (1988) Adenoviruses from patients with AIDS: a plethora of serotypes and a description of five new serotypes of subgenus D (types 43-47). J. Infect. Dis. 158, 804-813.

Hong, S.S., Karayan, L., Tournier, J., Curiel, D.T. and Boulanger, P.A. (1997) Adenovirus type 5 fiber knob binds to MHC class I (2 domain at the surface of human epithelial and B lymphoblastoid cells. EMBO J. 16: 2294-2306.

Hsu, K.H., Lonberg-Holm, K., Alstein, B. and Crowell, R.L. (1988)
A monoclonal antibody specific for the cellular receptor for the group B coxsackieviruses. J. Virol 62 (5): 1647-1652.

Huard, J., Lochmuller, H., Acsadi, G., Jani, A., Massie, B. and Karpati, G. (1995) The route of administration is a major determinant of the transduction efficiency of rat tissues by adenoviral recombinants. Gene Ther. 2: 107-115.

Ishibashi, M. and Yasue, H. (1984) The adenoviruses, H.S. Ginsberg, ed., Plenum Press, Londen, New York. Chapter 12, 497-561.

Iruela-Arispe, ML; Dvorak, HE (1997). Angiogenesis: a dynamic balance of stimulators and inhibitors. Thromb. Haemost 78, p672-677.

Kass-Eisler, A., Falck-Pederson, E., Elfenbein, D.H., Alvira, M., Buttrick, P.M. and Leinwand, L.A. (1994) The impact of developmental stage, route of administration and the immune system on adenovirus-mediated gene transfer. Gene Ther. 1: 395-402.

Khoo, S.H., Bailey, A.S., De Jong, J.C., and Mandal, B.K. (1995). Adenovirus infections in human immunodeficiency virus-positive patients: Clinical features and molecular epidemiology. J. Infect. Dis 172, 629-637

Kidd, A.H., Chroboczek, J., Cusack, S., and Ruigrok, R.W. (1993) Adenovirus type 40 virions contain two distinct fibers. Virology 192, 73-84.

Krasnykh V.N., Mikheeva G.V., Douglas J.T. and Curiel D.T. (1996) Generation of recombinant adenovirus vectors with modified fibers for altering viral tropism. J. Virol. 70(10): 6839-6846.

Krasnykh V.N., Dmitriev I., Mikheeva G., Miller C.R., Belousova N. and Curiel D.T. (1998) Characterization of an adenovirus vector containing a heterologous peptide epitope in the HI loop of the fiber knob. J. Virol. 72(3): 1844-1852.

Law, L., Chillon, M., Bosch, A., Armentano, D., Welsh, M.J. and Davidson, B.L. (1998) Infection of primary CNS cells by different adenoviral serotypes: Searching for a more efficient vector. Abstract 1^{st} Annual Meeting American Society of Gene Therapy, Seattle, Washington.

Loots, MA (1998). Differences in cellular infiltrate and extracellular matrix of chronic diabetic and venous ulcers versus acute wounds. J. Invest. Dermatol. 111, p850-857.

Leppard, K.N. (1997) E4 gene function in adenovirus, adenovirus vector and adeno-associated virus infections. J. Gen. Virol. 78: 2131-2138.

Machesney, M; Tidman, N; Waseem, A; Kirby, L;Leigh, I. (1998). Activated keratinocytes in the epidermis of hypertrophic scars. Am. J. Pathol. 152, p1133-1141.

Mignatti, P; Rifkin, DB; Welgus, HG; Parks, WC (1996). Proteinases and tissue remodeling. In: Clark RAF ed. The molecular and cellular biology of wound repair 2^{nd} ed. New York: Plenum Press p427-474.

Morgan, C., Rozenkrantz, H.S., and Mednis, B. (1969) Structure and development of viruses as observed in the electron microscope.X. Entry and uncoating of adenovirus. J. Virol 4, 777-796.

Pilcher, BK; Dumin, JA; Sudbeck, BD; Krane, SM; Welgus, HG; Parks, WC (1997). The activity of collagenase-1 is required for keratocyte migration on a type I collagen matrix. J. Cell. Biol. 137, p1445-1457.

Roelvink, P.W., Kovesdi, I. and Wickham, T.J. (1996) Comparative analysis of adenovirus fiber-cell interaction: Adenovirus type 2 (Ad2) and Ad9 utilize the same cellular fiber receptor but use different binding stratagies for attachemnt. J. Virol. 70: 7614-7621.

Roelvink, P.W., Lizonova, A., Lee, J.G.M., Li, Y., Bergelson, J.M., Finberg, R.W., Brough, D.E., Kovesdi, I. and Wickham, T.J. (1998) The coxsackie-adenovirus receptor protein can function as a cellular attachment protein for adenovirus serotypes from subgroups A, C, D, E, and F. J. Virol. 72: 7909-7915.

Roest, PAM; Van der Tuin, AC; Ginjaar, HB; Hoeben, RC; Hogervorst, FBL; Bakker, E; Den Dunnen, JT; Van Ommen, GJB (1996). Application of in vitro myo-differentiation of nonmuscle cells to enhance gene expression and facilitate analysis of muscle proteins. Neuromusc. Disord. 6, p195-202.

Roest, PAM; Bout, M; van der Tuin, AC; Ginjaar, IB; Bakker, E; Hogervorst, FBL; van Ommen, GJB; den Dunnen, JT (1996). Splicing mutations in DMD/BMD detected by RT-PCR/PTT: Detection of a 19AA insertion in the cysteine rich domain of dystrophin compatible with BMD.

Rogers, B.E., Douglas J.T., Ahlem, C., Buchsbaum, D.J., Frincke, J. and Curiel, D.T. (1997) Use of a novel crosslinking method to modify adenovirus tropism. Gene Ther.4: 1387-1392.

Risau, W (1997). Mechanisms of angiogenesis. Nature 386, p671-674.

Schulick, A.H., Vassalli, G., Dunn, P.F., Dong, G., Rade, J.J., Zamarron, C. and Dichek, D.A. (1997). Established immunity precludes adenovirus-mediated gene transfer in rat carotid arteries.

Schnurr, D and Dondero, M.E. (1993) Two new candidate adenovirus serotypes. Intervirol. 36, 79-83.

Shabram, P.W., Giroux, D.D., Goudreau, A.M., Gregory, R.J., Horn, M.T., Huyghe, B.G., Liu, X., Nunnally, M.H., Sugarman, B.J. and Sutjipto, S. (1997) Analytical anion-exchange HPLC of recombinant type-5 adenoviral particles. Hum. Gene Ther. 8 (4) : 453-465.

Singer, AJ; Clark, RAF (1999). Cutaneous wound healing. New. Eng. J. Med. 341, p738-746.

Saed, GM; Ladin, D; Olson, J. (1998). Analysis of p53 gene mutations in keloids using polymerase chain reaction-based single-strand conformational polymorphism and DNA sequencing. Arch. Dermatol. 134, p963-967.

Signas, G., Akusjarvi, G., and Petterson, U. (1985). Adenovirus 3 fiberpolypeptide gene: Complications for the structure of the fiber protein. J. Virol. 53, 672-678.

Stevenson S.C., Rollence M., White B., Weaver L. and McClelland A., (1995) Human adenovirus serotypes 3 and 5 bind to two different cellular receptors via the fiber head domain. J. Virol 69(5): 2850-2857.

Stevenson S.C., Rollence M., Marshall-Neff J. and McClelland A. (1997) Selective targeting of human cells by a chimaeric adenovirus vector containing a modified fiber protein. J. Virology 71(6): 4782-4790.

Stouten, P.W.F., Sander, C., Ruigrok, R.W.H., and Cusack, S. (1992) New triple helical model for the shaft of the adenovirus fiber. J. Mol. Biol. 226, 1073-1084.

Svensson, V. and Persson, R. (1984). Entry of adenovirus 2 into Hela cells. J. Virol. 51, 687-694.

Thivolet, J; Faure, M; Demidem, A; Mauduit, G (1986). Longterm survival and immunological tolerance of human epidermal allografts produced in culture. Transplantation 42, p274-280.

Tredget, EF; Nedelec, B; Scott, PG; Ghahary, A (1997). Hypertrophic scars, keyloids, and contractures: the cellular and molecular basis for therapy. Surg. Clin. North. Am. 77, p701-730.

Van der Vliet, P.C. (1995) Adenovirus DNA replication In: W. Doerfler and P. Böhm (eds.), The molecular repertoire of adenoviruses II. Springer-Verlag, Berlin.

Varga, M.J., Weibull, C., and Everitt, E. (1991). Infectious entry pathway of adenovirus type 2. J. Virol 65, 6061-6070.

Wadell G (1984) Molecular Epidemiology of human adenoviruses *Curr*. *Top. Microbiol.Immunol.* 110, 191-220.

Wickham T.J., Carrion M.E. and Kovesdi I., 1995. Targeting of adenovirus penton base to new receptors through replacement of its RGD motif with other receptor-specific peptide motifs. Gene Therapy **2:** 750-756.

Wickham, T.J., Mathias, P., Cherish, D.A., and Nemerow, G.R. (1993) Integrins avb3 and avb5 promote adenovirus internalization but not virus attachment. Cell 73, 309-319.

Wold, W.S., Tollefson, A.E. and Hermiston, T.W. (1995) E3 transcription unit of adenovirus. In: W. Doerfler and P. Böhm (eds.), The molecular repertoire of adenoviruses I. Springer-Verlag, Berlin.

Zabner, J., Armentano, D., Chillon, M., Wadsworth, S.C. and Welsh, M.J. (1998) Type 17 fiber enhances gene transfer Abstract 1^{st} Annual Meeting American Society of Gene Therapy, Seattle, Washington.

Zhang, K; Garner, W; Cohen, L; Rodrigues, J; Phan, S (1995). Increased types I and III collagen and transforming growth factor-beta 11 mRNA and protein in hypertrophic burn scar. J. Invest. Dermatol. 104, p750-754.

**Table 1**

| **Syndrom** | **Subgenus** | **Serotype** |
|---|---|---|
| Respiratory illness | A | 31 |
| | B | 3, 7, 11, 14, 21, 34, 35, 51 |
| | C | 1, 2, 5, 6 |
| | D | 39, 42-48 |
| | E | 4 |
| Keratoconjunctivitis (eye) | B | 11 |
| | D | 8, 19, 37, 50 |
| Hemorrhagic cystitis (Kidney) | B | 7, 11, 14, 16, 21, 34, 35 |
| And urogenital tract infections | C | 5 |
| | D | 39, 42-48 |
| Sexual transmission | C | 2 |
| | D | 19, 37 |
| Gastroenteritis | A | 31 |
| | B | 3 |
| | C | 1, 2, 5 |
| | D | 28 |
| | F | 40, 41 |
| CNS disease | A | 12, 31 |
| | B | 3,7 |
| | C | 2, 5, 6 |
| | D | 32, 49 |
| Hepatitis | A | 31 |
| | C | 1, 2, 5 |
| Disseminated | A | 31 |
| | B | 3,7,11,21 |
| | D | 30, 43-47 |
| None (???) | A | 18 |
| | D | 9, 10, 13, 15 17, 20, 22-29, 33, 36, 38 |

**Table 2**

| **type of skin substitute /brand name** | **Components** | **Advantages** | **Disadvantages** |
|---|---|---|---|
| **Epidermal** | Cultured autologous epidermal cells Cultured allogeneic Epidermal cells | Wide and permanent skin coverage Ready availibility, no need for biopsy | 2-3 week delay, high cost fragility, labor intensive use Temporary superficial coverage |
| | | | |
| **Dermal** | Cryopreserved allogeneic Skin | Ready availibility, use as base for cultured Epidermal cells | Need for procurement, potential disease transmission |
| Alloderm | Decellularized allogeneic Human skin | Ready availibility, inert nature, use as base For epidermal grafts | Need for procurement, potential disease transmission |
| Integra | Bovine collagen with Chondroitin-6-sulphate | Ready availibility, possible use of thin Autograft, reduced scarring | Need to excise wounds, risk of infection, high cost |
| Dermagraft-TC | Fibroblasts on nylon Mesh | Ready availibility, low reoccurence of ulcers | Possible need for multiple applications |

| **CombinedEpidermal/dermal** | | | |
|---|---|---|---|
| Appligraf | Bovine collagen, allogeneic Fibroblasts and epidermal Cells | Ready availibility, no need for subsequent autografting | Limited viability |
| Composite Cultured skin | Collagen matrix substrate with fibroblasts and Epidermal cells | Ready availibility, no need for subsequent autografting | Limited viability |

**Table 3**

| Adenovirus | Virus particles/ ml |
|---|---|
| Ad5Fib5 | 2.2 x 10¹² |
| Ad5Fib9 | 4.9 x 10¹¹ |
| Ad5Fib10 | 5.5 x 10¹¹ |
| Ad5Fib11 | 1.1 x 10¹² |
| Ad5Fib12 | 4.4 x 10¹² |
| Ad5Fib13 | 1.1 x 10¹² |
| Ad5Fib16 | 1.4 x 10¹² |
| Ad5Fib17 | 9.3 x 10¹¹ |
| Ad5Fib24 | 1.0 x 10¹² |
| Ad5Fib27 | 3.0 x 10¹¹ |
| Ad5Fib30 | 7.1 x 10¹¹ |
| Ad5Fib32 | 2.0 x 10¹² |
| Ad5Fib33 | 1.5 x 10¹² |
| Ad5Fib35 | 2.0 x 10¹² |
| Ad5Fib38 | 5.8 x 10¹¹ |
| Ad5Fib40-S | 3.2 x 10¹⁰ |
| Ad5Fib40-L | 2.0 x 10¹² |
| Ad5Fib45 | 2.8 x 10¹² |
| Ad5Fib47 | 2.6 x 10¹² |
| Ad5Fib49 | 1.2 x 10¹² |
| Ad5Fib51 | 5.1 x 10¹² |

## Claims

1. Use of a recombinant adenovirus having a tropism for human primary fibroblasts as a vehicle for delivering a nucleic acid of interest to a human primary fibroblast.

2. Use according to claim 1, in which said recombinant adenovirus is a chimaeric adenovirus.

3. Use according to claim 1 or 2, wherein said tropism is provided by at least a tropism determining part of an adenoviral fiber protein of a B-type or a D-type adenovirus.

4. Use according to claim 3, wherein said fiber protein is derived from an adenovirus type 11, 16, 35, 51, 9, 13, 17, 32 and/or 38.

5. Use according to claim 1 or 2, wherein said fiber protein is derived from a short fiber protein of an adenovirus type 40.

6. Use according to claim 4, wherein said fiber protein is derived from an adenovirus type 16 or a functional equivalent and/or homolog thereof.

7. Use according to any one of claims 1-6 wherein said nucleic acid of interest encodes a proteinaceous substance which improves angiogenesis and/or neovascularisation.

8. Use according to any one of claims 1-6 wherein said nucleic acid of interest is a MyoD gene and/or a Myogenin gene or a functional equivalent thereof.

9. Use according to any one of claims 2-8 wherein said chimaeric virus comprises an adenovirus 5 nucleic acid sequence.

10. Use according to claim 9, wherein said chimaeric virus comprises an adenovirus 5 genome, having at least a deletion in its E1 region where a nucleic acid of interest is inserted or can be inserted and a deletion in the fiber region which is replaced by a nucleic acid sequence having the desired tropism.

11. A gene delivery vehicle for delivering a nucleic acid of interest to a primary fibroblast, comprising a recombinant adenovirus having tropism for a primary fibroblast and a nucleic acid sequence encoding a proteinaceous substance which improves angiogenesis and/or neovascularisation as a nucleic acid of interest.

12. A gene delivery vehicle for delivering a nucleic acid of interest to a primary fibroblast, comprising a recombinant adenovirus having tropism for a primary fibroblast and a MyoD gene and/or a Myogenin gene or a functional equivalent thereof.

13. A gene delivery vehicle according to claim 11 or 12, wherein said tropism is provided by a nucleic acid sequence encoding at least a tropism determining part of a fiber protein of an adenovirus type B and/or adenovirus type D.

14. A gene delivery vehicle according to claim 11 or 12, wherein said tropism is provided by a nucleic acid sequence encoding at least a tropism determining part of a fiber protein of an adenovirus type 11, 16, 35, 51, 9, 13, 17, 32 and/or 38.

15. A gene delivery vehicle according to claim 14, wherein said tropism is provided by a nucleic acid sequence encoding at least a tropism determining part of a fiber protein of an adenovirus type 16.

16. A gene delivery vehicle according to any one of claims 11-15 wherein said recombinant adenovirus is a chimaeric adenovirus.

17. A gene delivery vehicle according to claim 16, wherein said chimaeric virus comprises an adenovirus 5 genome, having at least a deletion in its E1 region where a nucleic acid of interest is inserted or can be inserted and a deletion in the fiber region which is replaced by a nucleic acid sequence having the desired tropism.

18. A gene delivery vehicle according to any one of claims 11-17 for use as a pharmaceutical.

19. Use of a gene delivery according to any one of claims 11 or 13-17 in the preparation of a medicament for woundhealing and/or skin transplantation.

20. A human primary fibroblast transduced with a gene delivery vehicle according to any one of claims 11, or 13-17.

21. A skin allograft comprising a human primary fibroblast or offspring thereof according to claim 20.

22. A method for improving allogeneic or autologous skin transplantation comprising transducing human primary fibroblasts with a gene delivery vehicle according to claim 11 or 13-17, preparing a skin graft with said human primary fibroblasts and applying said graft to a patient.
